# EUROPEAN PATENT APPLICATION

(11) **EP 1 532 996 A1**
(43) Date of publication of application: **25.05.2005**
(21) Application number: 03388077.4
(22) Date of filing: 20.11.2003
(51) Int. Cl.: A61M 5/32, B21G 1/08, B23K 26/08

(54) **Non grinded needle tip-geometry for an injection needle**

(71) Applicant: NOVO NORDISK A/S, 2880 Bagsvaerd (DK)
(72) Inventor: Jakobsen, Henning, 8900 Randers (DK)

(57) **Abstract**

The present invention relates to an injection needle (1) for injecting or retracting fluid through the skin of a mammal body and more particularly to non-grinded needle tip-geometry for an injection needle (1) for a reduced penetration force and reduced bleeding. The injection needle (1) comprises of a needle shaft (8) having a centrally located bore disposed along a longitudinal axis (12) connected to a hub (9) by connecting means. The sharpened distal end (11) of the needle shaft (8) has an exit port (13) and two bevels (16,17) surrounding the exit port (13). The bevels (16,17) converge into a tip at the distal end to form a cutting edge (18) and at the proximal end into a heel (19). The bevels (16,17), cutting edge (18) and heel (19) together form the piercing edge that is sharp and continuous without any grinding transitions.

## Description

### THE TECHNICAL FIELD OF THE INVENTION:

The present invention relates to an injection needle for injecting or retracting fluid through the skin of a mammal body and more particularly to non-grinded needle tip-geometry for an injection needle for a reduced penetration force and reduced bleeding

This invention further relates to a method of sharpening the needle point.

### BACKGROUND OF INVENTION:

Conventional hypodermic needles typically include a hollow shaft having a fluid conducting lumen which is secured to a hub and a piercing part that is a more or less a sharply angled wedge at the distal end of the hypodermic needle. The hub is connected to a fluid delivery device such as a syringe such that the hollow shaft forms a channel from beneath the skin of the mammal body to the delivery device through which the fluid can be transported.

A hypodermic needle is typically used for performing the function of injecting or retracting drugs, medications fluid or like into the subcutaneous tissue.

Some drugs, such as insulin are self administered, and a typical diabetic person will require subcutaneous injections of insulin several times during the course of the day. Recent studies have indicated that people who inject themselves experience less pain when using a thin needle shaft i.e. a needle shaft having a little outside diameter. In order to reduce the discomfort of having to inject oneself several times a day, injection needles with a very thin needle shaft are preferred among people suffering from diabetes.

Besides the diameter of the needle, the geometry of the tip also influences on the pain perception. It is generally recognized that by reducing needle penetration force, the user will experience less pain, making the injection more comfortable.

Attempts have been made to minimize the needle penetration force necessary for urging the tip of the needle shaft through the skin of the user.

In US patent no. 3071135, which is incorporated as if fully set forth herein, the needle face is characterized by a pair of bevels which intersect with the main facet. The heel portion of the needle face includes an external recess, which merges with a smoothly rounded surface or edge portion of the lumen opening.

US patent No. 2409979 for a hypodermic needle is also intended to reduce the pain experienced by the patient as the needle penetrates the tissue.

In US patent No. 2560162, an additional pair of forward side bevels is provided. In this patent, the provision of the recess prevents or minimizes the severing of a plug from a layer (s) being pierced by the needle.

A five-beveled needle shaft is disclosed in US 3308822. The tip geometry disclosed is also made by grinding the distal end of the needle shaft. The needle tip is formed by grinding two additional bevels on traditional three-beveled needle tip geometry on the side away from the three bevels. The needle tip geometry of this patent greatly reduces penetration force and immediately following the initial penetration, the skin and underlying flesh is cleanly cut along three substantially straight lines radiating from the initial piercing point substantially in the form of the letter 'Y'.

A result of grinding, a multi beveled needle tip is a more continuous needle tip geometry though a high number of bevels tends to have a more sharp and delicate tip.

Another five-beveled needle tip geometry as disclosed in US 5752942 is an example of an attempt to lower the penetration force. The multi beveled needle-point includes a primary bevel, a pair of tip bevels and a pair of middle bevels. It has been surmised by the inventors that a primary reason that a patient experiences pain when the needle is inserted is that a portion of the needle point 'catches' on the skin or flesh as the needle penetrates. It has been reasoned that one cause of the needle catching the skin or flesh is due to the height of the 'intercept' established at the transition between different bevels forming the needle -point.

It is here believed that reducing the height of the intercept between the bevels making up the needle tip geometry will form a more continuous and unitary tip geometry that would require less penetration force.

In US patent nos. 3,308,822 and 5,752,942, the bevels are formed by conventional processes such as grinding.

When grinding such needle geometry, the skin-piercing tip of the needle shaft becomes extremely sharp pointed. Further, the grinding process results in transitions on the piercing edges that eventually affect the pain perception. Also, the said grinding transitions do not afford a uniform and continuous piercing edge. The ill effects of grinding are present on the needle tip.

A drawback with very sharp pointed tip is the possibility of accidental deforming the very tip either during the manufacturing of the injection needle or by the user when handling the injection needle.

Also, a very sharp needle and delicate tip such as the five-beveled tip geometry is more exposed to deformations than a not so sharp needle such as a three beveled needle tip geometry.

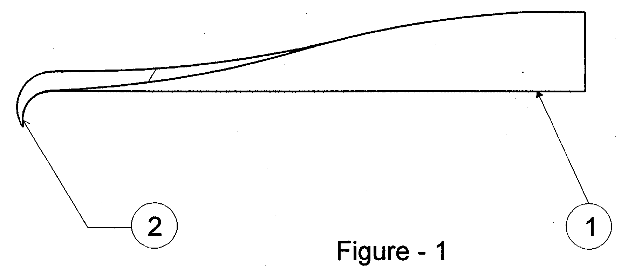

As shown in figure 1, a typical deformity called hook (2) can occur on the very tip of a sharp pointed needle (1) if the tip accidentally is bumped into an object. In the worst case the very tip can form a hook similar to what is known from the arrow shaped tip of a fishing hook. This influences strongly on the pain perception during injection since the hook on the needle tip will cause severe pain when removing the injection needle from the skin of the user.

### SUMMARY OF THE INVENTION

Accordingly, it is the object of the present invention to provide a new and an improved needle structure, which is capable of easier penetration into the patients tissue with minimum trauma with reduced bleeding

It is also an object of the present invention to provide for a needle structure with a robust needle point geometry. It is yet another object of the present invention to provide an injection needle with a piercing edge free from grinding transformations. A further object of the present invention is to provide a needle point geometry which is not too sharp pointed at the tip so as to avoid accidental deformation.

To overcome the drawbacks of the prior art the present invention provides for non-grinded needle tip for needles. Briefly described, a needle structure according to the present invention has a non-grinded tip which is cut by a laser and comprises of a needle shaft having a centrally located bore disposed along a longitudinal axis connected to a hub by connecting means. The sharpened distal end of the needle shaft has an exit port and two bevels surrounding the exit port. The bevels converge into a tip at the distal end to form a cutting edge and at the proximal end into a heel.

The bevels, cutting edge and heel together form the piercing edge that is sharp and continuous without any grinding transitions.

### BRIEF DESCRIPTION OF ACCOMPANYING DRAWINGS:

The accompanying drawings illustrate preferred embodiments of the invention and, together with the following detailed description, serve to explain the principles of the invention.
Figure 1 illustrates the tip of a sharp pointed needle with a typical deformity of conventional hypodermic needles.
Figure 2 illustrates multi-beveled needle tip geometry in accordance with prior art as shown in figure 5 of US patent 5752942.
Figure 3 illustrates a perspective view of a non-grinded needle tip in accordance with the present invention.
Figure 4 illustrate a sectional side view of an embodiment of a needle in accordance with the present invention.
Figure 5 illustrate a sectional side view of an alternate embodiment of a needle in accordance with the present invention.
Figure 6 illustrates a sectional side view of the distal end of a needle according to the invention.
Figure 7 illustrates a sectional view along the line A-A in figure 6.
Figure 8 illustrates a sectional top view of the needle in figure 3.
Figure 9 illustrates shows a sectional view along the line B-B in figure 6.
Figure 10 shows a schematic view of an embodiment of a method in accordance with the present invention .
Figure 11 shows a perspective of an embodiment of the needle in accordance with the present invention.
Figure 12 shows a sectional side view of the needle in figure 11.

### DETAILED DESCRIPTION OF THE DRAWINGS

It will be understood by those skilled in the art that the foregoing general description and the following detailed description are exemplary and explanatory of the invention and are not intended to be restrictive thereof.

Through out the patent specification, a convention employed is that in the appended drawings, like numerals denote like components.

At the outset it is appropriate to mention that the term 'distal' refers to a direction farthest from the practitioner and the term 'proximal' refers to a direction closest to the practitioner.

Referring first to Figure 1, there is shown a sharp pointed needle (1) with the formation of a hook (2) at the needle tip.

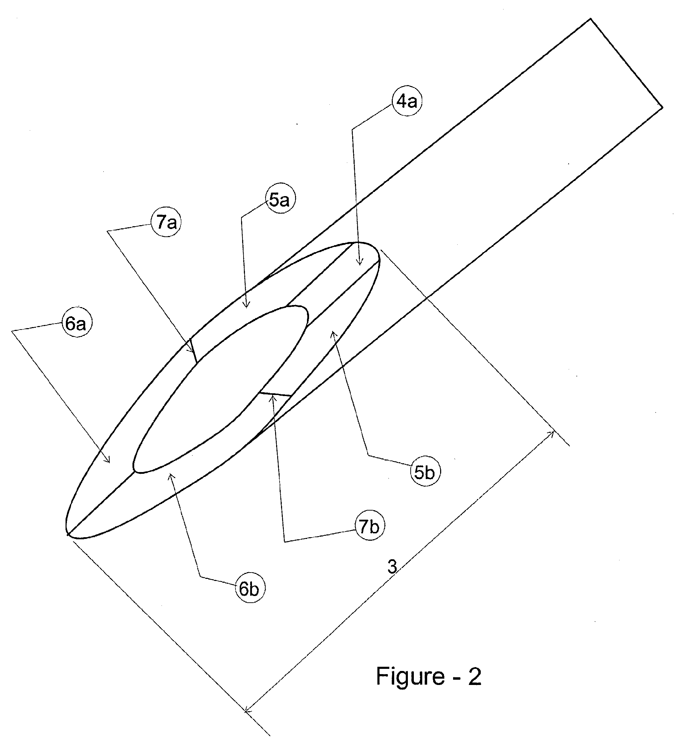

A multi-beveled needle tip geometry shown in Figure 2 in accordance with the prior art. The multi-beveled point (3) is formed through a plurality of individual bevels characterized by a primary bevel (4a), a pair of middle bevels (5a, 5b) and a pair of tip bevels (6a, 6b). The middle and tip bevels (5a, 6a) meet at an intersect (7a) demarcating the respective planes at which the middle and tip bevels are formed. Likewise, adjacent middle and tip bevels (5b, 6b) meet at intersect (7b).

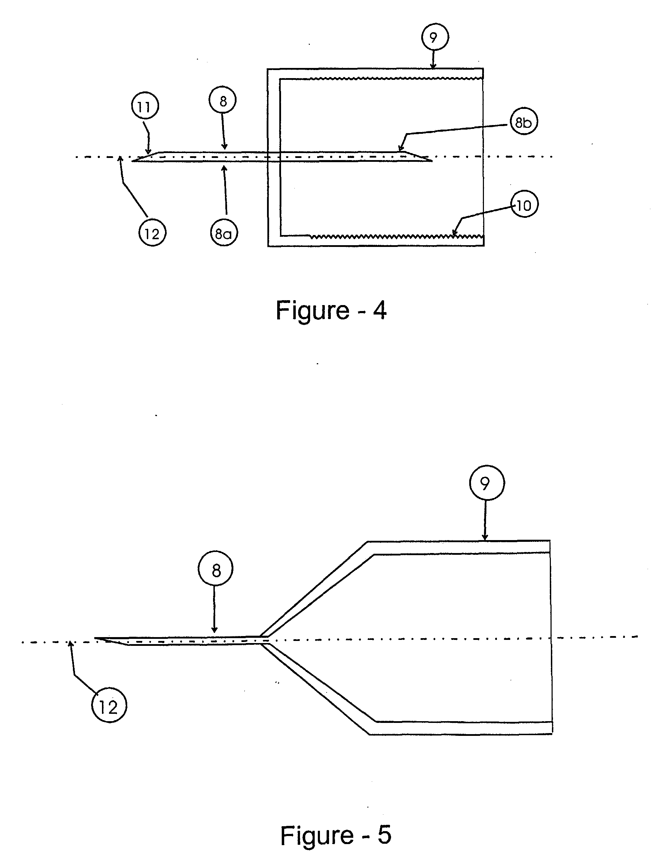

Turning to Figures 4 and 5, there is shown alternate embodiments for connecting the needle shaft to a hub. In figure 4, the needle shaft (8) is divided into a first part (8a) located outside the hub (9) for penetrating the skin of the mammal body and a second part (8b) located within the hub for penetrating a cartridge (not shown) containing the fluid to be injected. Further the hub (9) is provided with means (10) for connecting the hub to an injection pen. The first part (8a) is usually provided with a sharpened distal end (11) for providing an optimal penetration of the skin. The second part (8b) may be provided with a sharpened end as well.

Figure 5 discloses an alternate embodiment for connecting the needle shaft (8)to a somewhat different hub (9). This hub is particularly suitable for hypodermic syringes and is usually connected to the syringe by a traditional luer coupling.

The needle shaft (8) has a centrally located bore disposed along a central axis (12) for allowing fluid to flow through the needle shaft (8). The wall thickness of the needle shaft is defined as the difference between the diameter of the bore and the outside diameter of the needle shaft. ISO 9626 standard usually defines these dimensions.

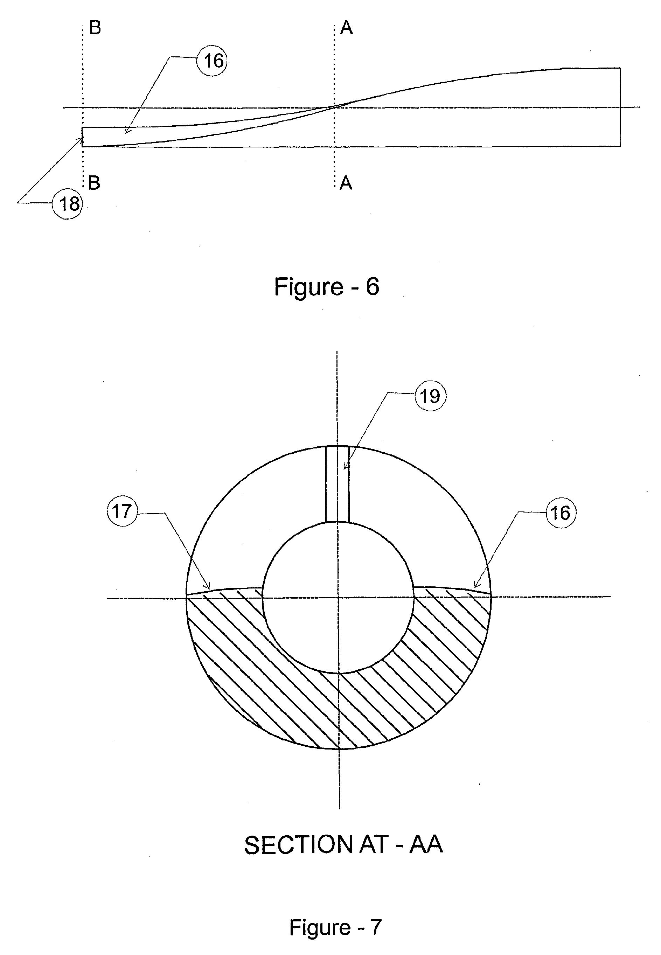

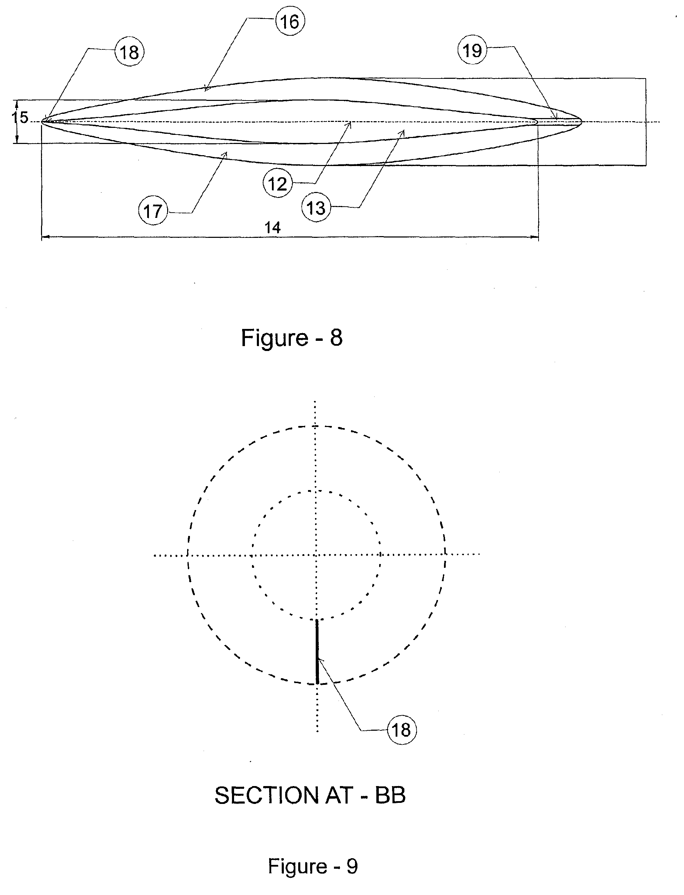

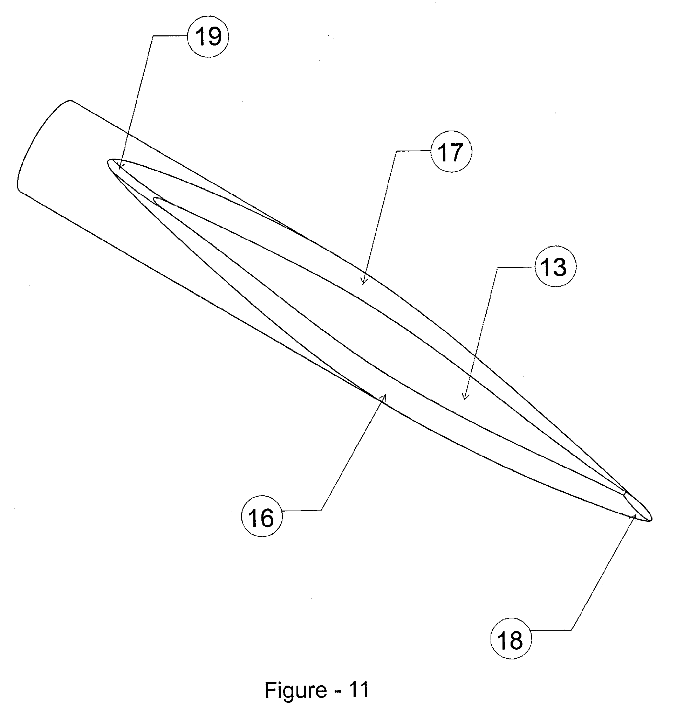

As shown in figures 6 and 8, the sharpened distal end (11) of the needle shaft (8) has an exit port (13) shaped as an ellipse with a major axis (14) parallel with the central axis (12) and a minor axis (15) perpendicular to the central axis (12). The exit port is surrounded by two bevels (16, 17) that converge into a tip formed as a cutting edge (18) at the most distal end of the major axis (14) and converge into a heel (19) at the opposite end of the longer axis (14) of the exit port (13). The exit port(13) thus formed comprises a cutting edge (18), a pair of bevels (16, 17) and a heel (19) which together form the piercing edge of the needle tip.

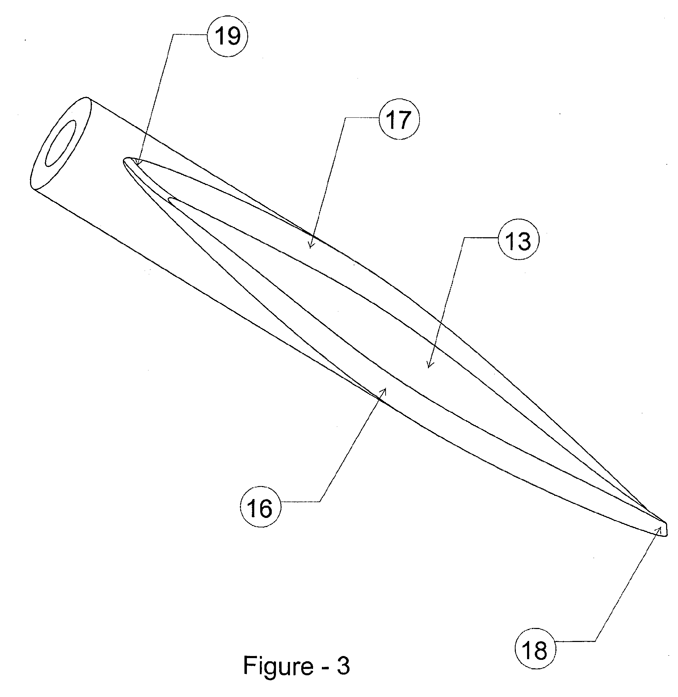

Laser is used for the cutting operation results which results in a uniform continuous and well-rounded piercing edge of the exit port. As can be seen from figures 3, 6 and 8, the cutting edge of the needle shaft though sharp and pointed affording a lower penetration force is also robust and less susceptible to accidental deformation. Further, the piercing edge is free from grinding transitions as occasioned by prior art.

Bevels 16 and 17 are equal in length and angle and are symmetrically formed on either side of the heel 19 and cutting edge 18.

The cutting edge as shown in figure 6 may be perpendicular to the central axis or may be an angle to the axis. The angle of inclination is preferably in the range of 45 and 90 degrees.

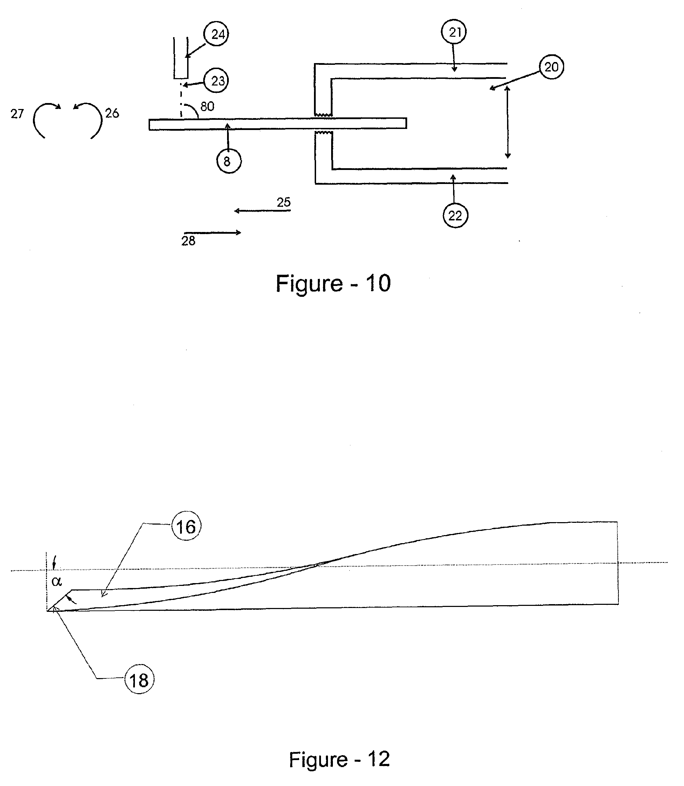

As shown in Figure 10, a non-grinded needle tip for injection can be made by securing the needle shaft in a movable tool (20) comprising a pair of jaws (21, 22) such that needle shaft moves with the tool. The tool is capable of longitudinal movement as well as rotation.

A laser beam (23) is directed from a laser emitter (24) onto the surface of the needle shaft (8). The laser beam (23) and the laser emitter (24) is locked in a stationary position throughout the cutting such that the laser beam (23) is perpendicular to the central axis (12) of the needle shaft (8). The impact angle between the laser beam (23) and the needle shaft (8) is 90 degrees.

Initially the laser beam (23) is directed onto the most distal end of the needle shaft (8). The laser beam (23) cuts through the wall of the needle shaft (8) forming an opening in the needle shaft (8). The movable tool (21) and the needle shaft (8) along with it is moved longitudinally parallel with the central axis of the bore in the direction indicated by the first arrow (25) (towards left as seen in the plane of the figure 9). During this longitudinal movement the needle shaft (8) is rotated. The rotation is first anti clockwise in the direction indicated by the arrow (26) until the shaft has been rotated 90 degrees to one side and then the rotation is clockwise for another 90 degrees as indicated by the arrow (27). This movement cuts the first bevel 16.

When the shaft is rotated back to its initial rotational position with the laser beam (23) impacting the surface of shaft at the most proximal end of the major axis (14), the needle shaft (8) is continuously rotated clockwise as indicated by the arrow (27). During this continued clockwise rotation the needle shaft (8) is moved longitudinally in the direction as indicated by the arrow (28) (towards right as seen in the plane of figure 9). Once the needle shaft has been rotated 90 degrees in the clockwise direction from initial position, the rotational direction is changed to anticlockwise and the laser beam (23) will encounter the initial cut at the most distal end of the major axis (14). This movement finalizes the second bevel (17). This predetermined movement of the movable tool and the needle shaft will form the exit port (13) with a smooth continuous piercing edge as disclosed in the figures 3, 6 and 8.

The two bevels (16, 17) meet at the most distal end of the major axis (14) as a cutting edge (18) and at the most proximal end as the heel (19). The cutting edge achieved by this laser cutting process is sharp, pointed and at the same time robust. The edges of the exit port and the cutting edge, together forming a piercing edge, realized by the above process are smooth, continuous, uniform and without grinding transformations. Such a needle tip offers better drug administration. Further, it is believed that the needle tip as disclosed in the preceding paragraphs results in reduced bleeding and also a reduced penetration force at the heel.

In an alternate embodiment, as shown in figures 11 and 12, the laser emitter (24) can be tilted during the movement of the needle shaft (8) such that the impact angle 80 can be different from 90 degrees. The laser emitter is preferably tilted when cutting at the most distal end of the major axis (14). In this way the cutting edge (18) can be provided at an angle 'α' with the central axis. The angle 'α' is preferably between 45 and 90 degrees. The opposite angle formed between the cutting edge (18) and an axis perpendicular to the longitudinal axis is preferably between 0 and 45 degrees. The angle 'α' preferably slopes towards the needle shaft 8 as disclosed in figure 4. In yet another embodiment the angle 'α' could slope away from the needle shaft 8.

It will readily be appreciated by those skilled in the art that the present invention is not limited to the specific embodiments herein shown. Thus variations may be made within the scope and spirit of the accompanying claims without sacrificing the principal advantages of the invention. Preferably only the very distal part of the tip needs to be made as a non-grinded tip in order to obtain a cutting edge (18). The proximal part end of the tip could be made in a traditional manner i.e. by grinding.

## Claims

1. An injection needle comprising:
- a needle shaft (8) having a centrally located bore disposed along a longitudinal axis (12) connected to a hub (9), **characterized in that**,
- the sharpened distal end of the needle shaft (8) has an exit port (13),
- two bevels (16, 17) surrounding the exit port (13),
- the bevels (16, 17) converge into a tip at the distal end to form a cutting edge (18) and at the proximal end into a heel (19), the cutting edge (18) being at an angle 'α' with the longitudinal axis (12),
- the bevels (16, 17), cutting edge (18) and heel (19) together form a piercing edge, wherein at least the tip part of the piercing edge is sharp and continuous without any grinding transitions.

2. An injection needle of claim 1, **characterized in that**, at least the tip part of the piercing edge is non-grinded.

3. An injection needle of claim 1 or 2, **characterized in that** the exit port (13) has a major axis (14) parallel to the longitudinal axis (12) and a minor axis (15) perpendicular to the longitudinal axis (12) of the needle shaft (8).

4. An injection needle of claim 1 to 3, **characterized in that** the angle 'α' is in the range of 45 ° to 90 °.

5. An injection needle of claim 1 to 3, **characterized in that** the angle formed between the cutting edge and the axis perpendicular to the longitudinal axis (12) is in the range of 0-45 °.

6. An injection needle of claim 4 or 5, **characterized in that**, the angle 'α' is approximately 90 °.

7. An injection needle of claim 6, **characterized in that**, the height of the cutting edge (18) substantially equals the wall thickness of the needle shaft (8).

8. A method of making an injection needle having a non-grinded tip comprising a pair of bevels (16, 17), a cutting edge (18) and a heel (19) forming an exit port (13), **characterized by** comprising the steps of:
- clamping the needle shaft (8) in a movable tool (20) that moves parallel to the longitudinal axis (12) of the needle shaft (8) and is capable of longitudinal as well as rotational movement,
- directing a laser beam (23) at the needle shaft (8) to make the first cut, and
- moving the needle shaft (8) in a predetermined pattern.

9. A method of claim 8, **characterized by** further comprising the steps of:
- longitudinally moving the needle shaft (8) in a first direction and simultaneously rotating the needle shaft by 90 degrees in a first rotational direction and in the opposite direction by 90 degrees such that the first bevel (16) is cut,
- longitudinally moving the needle shaft (8) in an opposite second direction while continuing rotation of the needle shaft (8) for another 90 degrees and in the first rotational direction for 90 degrees such that the laser beam (23) encounters the initial cut, thereby cutting the second bevel (17) and the cutting (18) edge between the first (16) and second (17) bevel at the distal end.

10. A method of claim 9, **characterized in that**, the laser beam (23) is inclined to the central axis (12) of the needle shaft (8).
